# DEMANDE DE BREVET EUROPEEN

(11) **EP 1 466 580 A1**
(43) Date de publication de la demande: **13.10.2004**
(21) Numéro de dépôt: 04290876.4
(22) Date de dépôt: 02.04.2004
(51) Int. Cl.: A61K 7/032

(54) **Composition cosmétique présentant un profil thermique particulier**

(30) Priorité: 11.04.2003 FR 0304593; 17.04.2003 FR 0304825
(71) Demandeur: L'OREAL, 75008 Paris (FR)
(72) Inventeur: De la Poterie, Valérie, 77820 Le Chatelet en Brie (FR)
(74) Mandataire: Kromer, Christophe

(57) **Abrégé**

La présente invention a pour objet une composition cosmétique comprenant :
i) un milieu physiologiquement acceptable,
ii) au moins un composé conférant à la composition un profil thermique présentant un pic de fusion dont la largeur à mi-hauteur Lf est inférieure ou égale à 20 °C,
ladite composition étant stable thermiquement.

La présente invention a également pour objet l'utilisation d'une telle composition pour obtenir un film déposé sur les fibres kératiniques homogène et/ou présentant des propriétés de recourbement améliorées.

## Description

La présente invention a pour objet une composition cosmétique ayant un profil thermique particulier qui présente un pic de fusion dont la largeur à mi-hauteur (Lf) est inférieure ou égale à 20°C, ladite composition étant stable thermiquement.
La composition selon l'invention peut être notamment une composition cosmétique de revêtement des fibres kératiniques telles que les cils, les sourcils et les cheveux d'êtres humains, ou bien encore des faux-cils. L'invention a également pour objet un procédé de maquillage ou de soin des matières kératiniques.

La composition selon l'invention peut être une composition de maquillage, encore appelée mascara, une base de maquillage des fibres kératiniques ou base-coat, une composition à appliquer sur un maquillage, dite encore top-coat, ou bien encore une composition de traitement des fibres kératiniques. Plus spécialement, la composition selon l'invention est un mascara.

Il est connu de l'art antérieur des compositions de mascara dites recourbantes comprenant un mélange de cires et de polymère filmogène telles que les compositions décrites dans le document EP-B-0928607.
On connaît également du document WO 00/74519 l'utilisation d'organogélateurs dans des compositions de mascara afin de remplacer tout ou partie des cires pour obtenir des propriétés de recourbement améliorées des cils.
Toutefois, de telles compositions ne permettent pas un recourbement optimale des cils.

D'autre part, il existe des dispositifs pour recourber les cils tels que les "recourbe-cils". Un type de recourbe-cils consiste par exemple à pincer les cils entre les machoires d'une pince pour leur conférer une forme courbée avant le maquillage mais cette opération est délicate à réaliser.
D'autres recourbe-cils se présentent sous la forme de pince ou de brosse chauffantes, tels que décrites dans les documents US-5853010 ou JP 2000-38314, pour une mise en forme du cil sous l'action de la chaleur. Ces instruments chauffants peuvent être appliqués sur des cils nus, mais l'effet recourbant obtenu est faible, ou sur des cils revêtus d'une composition de mascara quelconque. Dans ce dernier cas, il est fréquent qu'une partie de la composition de revêtement des cils soit éliminée sous l'action de la chaleur ou que les propriétés cosmétiques de cette composition soient dégradées (par exemple le film de composition perd son homogénéité, les cils se collent ensemble), ce qui conduit à un maquillage inesthétique des fibres kératiniques.

Le but de la présente invention est de disposer d'une composition de revêtement des fibres kératiniques permettant un recourbement amélioré des cils, notamment sous l'action de la chaleur, et présentant de bonnes propriétés cosmétiques en particulier un dépôt homogène et une bonne tenue du recourbement dans le temps.
Cette composition peut être notamment utilisée en association avec un instrument chauffant, tel qu'une brosse chauffante, qui peut être appliquée sur les cils avant, pendant ou après que ceux-ci soient revêtus de la composition ou conditionnée dans un dispositif permettant d'appliquer la composition à chaud.

Les inventeurs ont découvert qu'un mascara présentant les propriétés décrites ci-dessus peut être obtenu en utilisant une composition présentant un profil thermique particulier.

De façon plus précise, l'invention a pour objet une composition cosmétique comprenant :
i) un milieu physiologiquement acceptable et
ii) au moins un composé conférant à ladite composition un profil thermique présentant un pic de fusion dont la largeur à mi-hauteur Lf est inférieure ou égale à 20 °C,
ladite composition étant stable thermiquement.

Par «milieu physiologiquement acceptable », on entend un milieu non toxique et susceptible d'être appliqué sur les fibres kératiniques, telles que les cils, les sourcils et les cheveux d'êtres humains, notamment compatible avec la zone oculaire.

Par composition "stable thermiquement" on désigne une composition dont la viscosité varie d'au plus 25 % et de préférence, d'au plus 20 %, et de préférence encore, d'au plus 15 %, et de préférence encore, d'au plus 10 %, après avoir été soumise à une succession de X cycles de fusion/refroidissement selon le protocole suivant : On dispose la composition dans une étuve à 80 °C, pendant 2 heures. On laisse ensuite la composition revenir naturellement à température ambiante. On en mesure la viscosité après mise en oeuvre des X cycles. Entre deux cycles successifs, on laisse 24h. La viscosité mesurée après mise en oeuvre des X cycles de fusion/refroidissement est comparée avec celle mesurée avant le premier cycle.

De préférence, X = 4 . De préférence encore, X = 8. De préférence encore X = 10. De préférence encore, X = 15.

En raison de cette caractéristique de stabilité thermique, les propriétés cosmétiques de la composition ne sont pas altérées de manière sensible par les changements de température subis lorsqu'elle est soumise à une source de chaleur.

La présente invention a également pour objet l'utilisation d'une composition cosmétique de revêtement des fibres kératiniques comprenant :
i) un milieu physiologiquement acceptable et
ii) au moins un composé conférant à ladite composition un profil thermique présentant un pic de fusion dont la largeur à mi-hauteur Lf est inférieure ou égale à 20 °C,
, ladite composition étant stable thermiquement, pour obtenir un film déposé sur les fibres kératiniques homogène et/ou présentant des propriétés de recourbement améliorées.

L"invention a également pour objet un procédé cosmétique de maquillage ou de soin non thérapeutique des fibres kératiniques comprenant l'application sur les fibres kératiniques d'une composition telle que définie ci-dessus.

L'invention a encore pour objet un procédé cosmétique de maquillage ou de soin non thérapeutique des matières kératiniques (telles que la peau, les lèvres ou les fibres kératiniques) comprenant l'application sur les matières kératiniques d'une composition cosmétique telle que décrite ci-dessus, ladite composition étant, antérieurement, simultanément, ou postérieurement à son application, portée à une température supérieure ou égale à son point de fusion Pf, de préférence supérieure ou égale à sa température de fin de fusion Tf.
La composition peut être portée à une température supérieure ou égale à son point de fusion préalablement à son application, notamment par chauffage au micro-ondes ou au bain-marie, ou par tout autre moyen de chauffage équipant un ensemble de conditionnement et d'application la contenant, en particulier, les parois d'un réservoir contenant la composition. Alternativement, la composition peut être portée à une température supérieure ou égale à son point de fusion simultanément ou postérieurement à son application, notamment à l'aide d'un dispositif d'application comprenant des moyens de chauffage, tel qu'une brosse chauffante.

### Détermination du profil thermique de la composition

Le profil thermique de la composition selon l'invention est réalisé à l'aide d'un calorimètre à balayage différentiel (D.S.C.), par exemple le calorimètre vendu sous la dénomination DSC 30 par la société METLER.
Un échantillon de 5 à 10 mg de produit disposé dans un creuset est soumis à une première montée en température allant de -20 °C à 90 °C, à la vitesse de chauffe de 5 °C/ minute, puis est refroidi de 90 °C à -20 °C à une vitesse de refroidissement de 5 °C/minute et enfin soumis à une deuxième montée en température allant de -20 °C à 90 °C à une vitesse de chauffe de 5 °C/minute. Pendant la deuxième montée en température, on mesure la variation de la différence de puissance absorbée par le creuset vide et par le creuset contenant l'échantillon de produit en fonction de la température.

Le profil thermique de la composition selon l'invention présente un pic de fusion étroit, il présente en particulier une largeur de pic à mi-hauteur Lf inférieure ou égale à 20°C, de préférence allant de 0,5°C à 20°C, mieux, de 1 °C à 10°C et mieux allant de 2°C à 5°C. La mi-hauteur du pic de fusion peut être déterminée sur la base de la demi distance entre une droite reliant deux portions planes du profil thermique de part et d'autre du pic de fusion, et le sommet du pic.

Le pic de fusion peut également présenter un point de fusion Pf allant de 20°C à 80°C, de préférence allant de 25°C à 75°C et mieux de 35°C à 60°C. Le point de fusion du pic considéré est la valeur de la température correspondant au sommet du pic de la courbe représentant la variation de la différence de puissance absorbée en fonction de la température.

Le pic de fusion peut en particulier présenter une faible amplitude de température ΔT = Tf - To inférieure ou égale à 30°C, de préférence allant de 1°C à 30°C, mieux de 2 °C à 25°C et mieux allant de 3°C à 20°C, To étant la température de début de fusion correspondant à la température mesurée lorsque 5% de l'enthalpie de fusion est consommée et Tf étant la température de fin de fusion qui correspond à la température mesurée lorsque 95% de l'enthalpie de fusion a été consommée.
De préférence, la température de début de fusion de la composition To est supérieure ou égale à 10°C, elle va par exemple de 10°C à 50°C, mieux supérieure ou égale à 15°C, par exemple allant de 15°C à 45°C et mieux encore, supérieure ou égale à 20°C, par exemple allant de à 20°C à 40°C.
Avantageusement, la température de fin de fusion de la composition Tf est inférieure ou égale à 90°C, de préférence allant de 35°C à 90°C, mieux inférieure ou égale à 80°C, par exemple allant de 40°C à 80°C et mieux encore, inférieure ou égale à 70°C, par exemple allant de à 40°C à 70°C et encore mieux, allant de 40 à 60°C.

Les compositions ayant de telles caractéristiques (notamment de faible amplitude ΔT) présentent la particularité, lorsqu'elles sont chauffées à une température supérieure à leur point de fusion Pf, de préférence à une température supérieure ou égale à leur température de fin de fusion (Tf), de passer d'un état amorphe (température supérieure à Pf) à un état cristallin (température inférieure à Pf) en un temps très court,
Ainsi, il est possible de procéder à une mise en forme des cils revêtus d'une telle composition sous l'action d'une source de chaleur (telle qu'une brosse chauffante) présentant une température supérieure ou égale au point de fusion de la composition, et ce rapidement.

De préférence, le profil thermique de la composition selon l'invention présente un pic de fusion unique.

De préférence, le composé conférant à la composition un profil thermique présentant un pic de fusion dont la largeur à mi-hauteur Lf est inférieure ou égale à 20 °C a lui -même un profil thermique présentant un pic de fusion dont la largeur à mi-hauteur Lf est inférieure ou égale à 20 °C. Ce composé est avantageusement choisi parmi les cires, les polymères et en particulier les polymères semi-cristallins, les huiles épaissies par un agent structurant et leurs mélanges.
Ce composé peut être présent en une teneur allant de 1% à 60%, de préférence de 3% à 55%, mieux de 5% à 50% et encore mieux de 10 à 40% en poids par rapport au poids total de la composition.

Dans la présente demande, une cire est un composé lipophile, solide à température ambiante (25 °C), à changement d'état solide/liquide réversible, ayant un point de fusion supérieur ou égal à 30 °C pouvant aller jusqu'à 120 °C. En portant la cire à l'état liquide (fusion), il est possible de la rendre miscible aux huiles et de former un mélange homogène microscopiquement, mais en ramenant la température du mélange à la température ambiante, on obtient une recristallisation de la cire dans les huiles du mélange.

Le point de fusion de la cire peut être mesuré à l'aide d'un calorimètre à balayage différentiel (D.S.C.), par exemple le calorimètre vendu sous la dénomination DSC 30 par la société METLER. Un échantillon de 15 mg de produit disposé dans un creuset est soumis à une première montée en température allant de 0°C à 120°C, à la vitesse de chauffe de 10°C/ minute, puis est refroidi de 120°C à 0°C à une vitesse de refroidissement de 10°C/minute et enfin soumis à une deuxième montée en température allant de 0°C à 120°C à une vitesse de chauffe de 5°C/minute. Pendant la deuxième montée en température, on mesure la variation de la différence de puissance absorbée par le creuset vide et par le creuset contenant l'échantillon de produit en fonction de la température. Le point de fusion du composé est la valeur de la température correspondant au sommet du pic de la courbe représentant la variation de la différence de puissance absorbée en fonction de la température.

Les cires susceptibles d'être utilisées dans la composition selon l'invention peuvent être choisies parmi les cires, solides et rigides à température ambiante d'origine animale, végétale, minérale ou de synthèse et leurs mélanges. Les cires peuvent avoir un point de fusion allant de 30 °C à 80 °C environ, de préférence allant de 30°C à 70°C et mieux allant de 35°C à 65°.

De préférence, la cire est choisie parmi la cire d'olive obtenue par hydrogénation d'huile d'olive estérifiée avec l'alcool stéarylique telle que la Phytowax Olive 18L57 (point de fusion Pf = 58,6°C) commercialisée par la société Sophim, l'alcool stéarique (point de fusion Pf = 60°C), le stéarate de stéaryle (point de fusion Pf = 57°C), le benzoate de stéaryle (point de fusion Pf = 40°C), le ditriméthylolpropanetetrastéarate (point de fusion Pf = 46°C), la cire Licowax KST (acides gras de cire de Montan polyéthoxylés) de la société Clariant (point de fusion Pf = 55°C), le ditriméthylolpropanetetrabéhénate (point de fusion Pf = 67,5°C), la cire de dioctadecylcarbonate (point de fusion Pf = 57°C) et leurs mélanges.

La composition selon l'invention peut comprendre un polymère. De préférence ce polymère est un polymère semi-cristallin.
Par "polymère semi-cristallin", on entend des polymères comportant une partie cristallisable, une chaîne pendante cristallisable ou une séquence cristallisable dans le squelette, et une partie amorphe dans le squelette et présentant une température de changement de phase réversible du premier ordre, en particulier de fusion (transition solide-liquide). Lorsque la partie cristallisable est sous forme d'une séquence cristallisable du squelette polymérique, la partie amorphe du polymère est sous forme de séquence amorphe ; le polymère semi-cristallin est dans ce cas un copolymère séquencé par exemple du type dibloc, tribloc ou multibloc, comportant au moins une séquence cristallisable et au moins une séquence amorphe. Par "séquence", on entend généralement au moins 5 motifs de répétition identiques. La ou les séquences cristallisables sont alors de nature chimique différente de la ou des séquences amorphes.

Le polymère semi-cristallin utilisable dans la composition selon l'invention a un point de fusion supérieur ou égal à 20°C (notamment allant de 20°C à 80°C), de préférence allant de 30°C à 70°C et mieux de 35°C à 65°C. Ce point de fusion est une température de changement d'état du premier ordre.
Le point de fusion peut être mesurée par toute méthode connue et en particulier à l'aide d'un calorimètre à balayage différentiel (D.S.C) tel que décrit plus haut.

De façon avantageuse, le ou les polymères semi-cristallins auxquelles s'applique l'invention présentent une masse moléculaire moyenne en nombre supérieure ou égale à 1 000.

De façon avantageuse, le ou les polymères semi-cristallins de la composition de l'invention ont une masse moléculaire moyenne en nombre Mn allant de 2 000 à 800 000, de préférence de 3 000 à 500 000, mieux de 4 000 à 150 000, notamment inférieure à 100 000, et mieux de 4 000 à 99 000. De préférence, ils présentent une masse moléculaire moyenne en nombre supérieure à 5 600, allant par exemple de 5 700 à 99 000.

Par "chaîne ou séquence cristallisable", on entend au sens de l'invention une chaîne ou séquence qui si elle était seule passerait de l'état amorphe à l'état cristallin, de façon réversible, selon qu'on est au-dessus ou en dessous de la température de fusion. Une chaîne au sens de l'invention est un groupement d'atomes, pendant ou latéral par rapport au squelette du polymère. Une séquence est un groupement d'atomes appartenant au squelette, groupement constituant un des motifs répétitif du polymère. Avantageusement, la "chaîne pendante cristallisable" peut être une chaîne comportant au moins 6 atomes de carbone.

De préférence, la ou les séquences ou chaînes cristallisables des polymères semi-cristallins représentent au moins 30 % du poids total de chaque polymère et mieux au moins 40 %. Les polymères semi-cristallins de l'invention à séquences cristallisables sont des polymères, séquencés ou multiséquencés. Ils peuvent être obtenus par polymérisation de monomère à double liaisons réactives (ou éthyléniques) ou par polycondensation. Lorsque les polymères de l'invention sont des polymères à chaînes latérales cristallisables, ces derniers sont avantageusement sous forme aléatoire ou statistique.

De préférence, les polymères semi-cristallins utilisables dans la composition selon l'invention sont d'origine synthétique. En outre, ils ne comportent pas de squelette polysaccharidique. De façon générale, les motifs (chaînes ou séquences) cristallisables des polymères semi-cristallins selon l'invention, proviennent de monomère(s) à séquence(s) ou chaîne(s) cristallisable(s), utilisé(s) pour la fabrication des polymères semi-cristallins.

Les polymères semi-cristallins utilisables dans la composition selon l'invention sont en particulier :
- les copolymères séquencés de polyoléfines à cristallisation contrôlée, notamment ceux dont les monomères sont décrits dans EP-A-0 951 897,
- les polycondensats et notamment de type polyester, aliphatique ou aromatique ou copolyester aliphatique/aromatique,
- les homo- ou co-polymères portant au moins une chaîne latérale cristallisable et les homo- ou co-polymères portant dans le squelette au moins une séquence cristallisable, comme ceux décrits dans le document US-A-5 156 911,
- les homo- ou co-polymères portant au moins une chaîne latérale cristallisable en particulier à groupement(s) fluoré(s) tels que décrits dans le document WO-A-01/19333,
et leurs mélanges. Dans ces deux derniers cas, la ou les chaînes latérales ou séquences cristallisables sont hydrophobes.

### A) Polymères semi-cristallins à chaînes latérales cristallisables

On peut citer en particulier ceux définis dans le document US-A-5156911 et WO-A-01/19333. Ce sont des homopolymères ou copolymères comportant de 50 à 100 % en poids de motifs résultant de la polymérisation d'un ou plusieurs monomères porteurs de chaîne latérale hydrophobe cristallisable.
. Ces homo- ou co-polymères sont de toute nature du moment qu'ils présentent les conditions indiquées précédemment.
Ils peuvent résulter :
- de la polymérisation notamment radicalaire d'un ou plusieurs monomères à double(s) liaison(s) réactive(s) ou éthyléniques vis-à-vis d'une polymérisation, à savoir à groupe vinylique, (méth)acrylique ou allylique.
- de la polycondensation d'un ou plusieurs monomères porteurs de groupes co-réactifs (acide carboxylique ou sulfonique, alcool, amine ou isocyanate), comme par exemple les polyesters, les polyuréthanes, les polyéthers, les polyurées, les polyamides.

D'une façon générale, ces polymères sont choisis notamment parmi les homopolymères et copolymères résultant de la polymérisation d'au moins un monomère à chaîne(s) cristallisable(s) qui peut être représenté par la formule X : 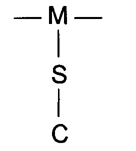 avec M représentant un atome du squelette polymérique, S représentant un espaceur, C représentant un groupe cristallisable.

Les chaînes « -S-C » cristallisables peuvent être aliphatiques ou aromatiques, éventuellement fluorées ou perfluorées. « S » représente notamment un groupe (CH₂)ₙ ou (CH₂CH₂O)ₙ ou (CH₂O), linéaire ou ramifié ou cyclique, avec n entier allant de 0 à 22. De préférence « S » est un groupe linéaire. De préférence, « S » et « C » sont différents.

Lorsque les chaînes « -S-C » cristallisables sont des chaînes aliphatiques hydrocarbonées, elles comportent des chaînes alkyle hydrocarbonées à au moins 11 atomes de carbone et au plus 40 atomes de carbone et mieux au plus 24 atomes de carbone. Il s'agit notamment de chaînes aliphatiques ou chaînes alkyle possédant au moins 12 atomes de carbone et de préférence, il s'agit de chaînes alkyles en C₁₄-C₂₄. Lorsqu'il s'agit de chaînes alkyle fluorées ou perfluorées, elles comportent au moins 6 atomes de carbone fluorés et notamment au moins 11 atomes de carbone dont au moins 6 atomes de carbone sont fluorés.

Comme exemple de polymères ou copolymères semi-cristallins à chaîne(s) cristallisable(s), on peut citer ceux résultant de la polymérisation d'un ou plusieurs monomères suivants : les (méth)acrylates d'alkyle saturés avec le groupe alkyle en C₁₄-C₂₄, les (méth)acrylates de perfluoroalkyle avec un groupe alkyle perfluoro en C₁₁-C₁₅, les N-alkyl (méth)acrylamides avec le groupe alkyle en C₁₄ à C₂₄ avec ou sans atome de fluor, les esters vinyliques à chaînes alkyle ou perfluoro (alkyle) avec le groupe alkyle en C₁₄ à C₂₄ (avec au moins 6 atomes de fluor pour une chaîne perfluoro alkyle), les éthers vinyliques à chaînes alkyle ou perfluoro (alkyle) avec le groupe alkyle en C₁₄ à C₂₄ et au moins 6 atomes de fluor pour une chaîne perfluoro alkyle, les alpha-oléfines en C₁₄ à C₂₄ comme par exemple l'octadécène, les para-alkyl styrènes avec un groupe alkyle comportant de 12 à 24 atomes de carbone, leurs mélanges.

Lorsque les polymères résultent d'une polycondensation, les chaînes cristallisables hydrocarbonées et/ou fluorées telles que définies ci-dessus, sont portées par un monomère qui peut être un diacide, un diol, une diamine, un di-isocyanate.

Lorsque les polymères sont des copolymères, ils contiennent, en plus, de 0 à 50% de groupes Y ou Z résultant de la copolymérisation :
α) de Y qui est un monomère polaire ou non polaire ou un mélange des deux :
   . Lorsque Y est un monomère polaire, c'est soit un monomère porteur de groupes polyoxyalkylénés (notamment oxyéthyléné et/ou oxypropyléné), un (méth)acrylate d'hydroxyalkyle comme l'acrylate d'hydroxyéthyle, le (méth)acrylamide, un N-alkyl(méth)acrylamide, un N,N-dialkyl(méth)acrylamide comme par exemple le N,N-diisopropylacrylamide ou la N-vinyl-pyrolidone (NVP), le N-vinyl caprolactame, un monomère porteur d'au moins un groupe acide carboxylique comme les acides (méth)acryliques, crotonique, itaconique, maléique, fumarique ou porteur d'un groupe anhydride d'acide carboxylique comme l'anhydre maléique, et leurs mélanges.
   . Lorsque Y est un monomère non polaire il peut être un ester du type (méth)acrylate d'alkyle linéaire ramifié ou cyclique, un ester vinylique, un alkyl vinyl éther, une alpha-oléfine, le styrène ou styrène substitué par un groupe alkyle en C₁ à C₁₀, comme l'α-méthylstyrène, un macromonomère du type polyorganosiloxane à insaturation vinylique.
      Par "alkyle», on entend au sens au sens de l'invention un groupement saturé notamment en C₈ à C₂₄, sauf mention exprès, et mieux en C₁₄ à C₂₄.
β) de Z qui est un monomère polaire ou un mélange de monomères polaires. Dans ce cas, Z a la même définition que le "Y polaire" défini ci-dessus.
   De préférence, les polymères semi-cristallins à chaîne latérale cristallisable sont des homopolymères d'alkyl(méth)acrylate ou d'alkyl(méth)acrylamide avec un groupe alkyle tel que défini ci-dessus, et notamment en C₁₄-C₂₄, des copolymères de ces monomères avec un monomère hydrophile de préférence de nature différente de l'acide (méth)acrylique comme la N-vinylpyrrolidone ou l'hydroxyéthyl (méth)acrylate et leurs mélanges.

### B) Les polymères portant dans le squelette au moins une séquence cristallisable

Ces polymères sont notamment des copolymères séquencés constitués d'au moins 2 séquences de nature chimique différente dont l'une est cristallisable.
- On peut utiliser les polymères séquencés définis dans le brevet US-A-5 156 911 ;
- Les copolymères séquencés d'oléfine ou de cyclooléfine à chaîne cristallisable comme ceux issus de la polymérisation séquencée de :
   . cyclobutène, cyclohexène, cyclooctène, norbornène (c'est-à-dire bicyclo(2,2,1)heptène-2), 5-méthylnorbornène, 5-éthylnorbornène, 5,6-diméthylnorbornène, 5,5,6-triméthyl norbornène, 5-éthylidène-norbornène, 5-phényl-norbonène, 5-benzylnorbornène, 5-vinyl norbornène, 1,4,5,8-diméthano-1,2,3,4,4a,5,8a-octahydronaphtalène, dicyclopentadiène ou leurs mélanges,
   . avec l'éthylène, le propylène, le 1-butène, le 3-méthyl-1-butène, le 1-hexène, le 4-méthyl-1-pentène, le 1-octène, le 1-décène, le 1-éicosène ou leurs mélanges,
   . et en particulier les copoly(éthylène/norbornène) blocs et les terpolymères (éthylène/propylène/éthylidène-norbornène) blocs. On peut aussi utiliser ceux résultants de la copolymérisation séquencée d'au moins 2 α-oléfines en C₂-C₁₆ et mieux en C₂-C₁₂ et encore mieux en C₄-C₁₂ tels que ceux cités précédemment et en particulier les bipolymères séquencés d'éthylène et d'1-octène.
- Les copolymères peuvent être des copolymères présentant au moins une séquence cristallisable, le reste du copolymère étant amorphe (à température ambiante). Ces copolymères peuvent, en outre, présenter deux séquences cristallisables de nature chimique différente. Les copolymères préférés sont ceux qui possèdent à la fois à température ambiante, une séquence cristallisable et une séquence amorphe à la fois hydrophobe et lipophile réparties séquentiellement ; on peut citer par exemple les polymères possédant une des séquences cristallisables et une des séquences amorphes suivantes :
   . Séquence cristallisable par nature : a) polyester comme les poly(alkylène téréphtalate), b) polyoléfine comme les polyéthylènes ou polypropylènes.
   . Séquence amorphe et lipophile comme les polyoléfines ou copoly(oléfine)s amorphes telles que le poly(isobutylène), le polybutadiène hydrogéné, le poly(isoprène) hydrogéné.

Comme exemple de tels copolymères à séquence cristallisable et à séquence amorphe distinctes, on peut citer :
α) les copolymères séquencés poly(ε-caprolactone)-b-poly(butadiène), utilisés de préférence hydrogénés, tels que ceux décrits dans l'article "Melting behavior of poly(ε-caprolactone)-block-polybutadiène copolymers" de S. Nojima, Macromolécules, 32, 3727-3734 (1999).
β) les copolymères séquencés poly(butylènetéréphtalate)-b-poly(isoprène) hydrogénés séquencés ou multiséquencés, cités dans l'article "Study of morphological and mechanical properties of PP/PBT" de B. Boutevin et al., Polymer Bulletin, 34, 117-123 (1995).
γ) les copolymères séquencés poly(éthylène)-b-copoly(éthylène/propylène) cités dans les articles "Morphology of semi-crystalline block copolymers of ethylene-(ethylene-alt-propylene)" de P. Rangarajan et al., Macromolecules, 26, 4640-4645 (1993) et " Polymer agregates with crystalline cores : the system poly(ethylene)-poly(ethylene-propylene)" de P. Richter et al., Macromolécules, 30, 1053-1068 (1997).
δ) les copolymères séquencés poly(éthylène)-b-poly(éthyléthylène) cités dans l'article général "Cristallization in block copolymers" de I.W. Hamley, Advances in Polymer Science, vol 148, 113-137 (1999).

Les polymères semi-cristallins de la composition de l'invention peuvent être ou non réticulés en partie du moment que le taux de réticulation ne gène pas leur dissolution ou dispersion dans la phase grasse liquide éventuellement présente dans la composition par chauffage au-dessus de leur température de fusion. Il peut s'agir alors d'une réticulation chimique, par réaction avec un monomère multifonctionnel lors de la polymérisation. Il peut aussi s'agir d'une réticulation physique qui peut alors être due soit à l'établissement de liaisons type hydrogène ou dipolaire entre des groupes portés par le polymère comme par exemple les interactions dipolaires entre ionomères carboxylates, ces interactions étant en faible quantité et portées par le squelette du polymère ; soit à une séparation de phase entre les séquences cristallisables et les séquences amorphes, portées par le polymère.

De préférence, les polymères semi-cristallins de la composition selon l'invention sont non réticulés.

Selon un mode particulier de réalisation de l'invention, le polymère est choisi parmi les copolymères résultant de la polymérisation d'au moins un monomère à chaîne cristallisable choisi parmi les (méth)acrylates d'alkyle saturés en C₁₄ à C₂₄, les (méth)acrylates de perfluoroalkyle en C₁₁ à C₁₅, les N alkyl (méth)acrylamides en C₁₄ à C₂₄ avec ou sans atome de fluor, les esters vinyliques à chaînes alkyle ou perfluoroalkyle en C₁₄ à C₂₄, les éthers vinyliques à chaînes alkyle ou perfluoroalkyle en C₁₄ à C₂₄, les alphaoléfines en C₁₄ à C₂₄, les para-alkyl styrènes avec un groupe alkyle comportant de 12 à 24 atomes de carbone, avec au moins un ester ou amide d'acide monocarboxylique en C₁ à C₁₀ fluoré ou non fluoré, qui peut être représenté par la formule suivante : 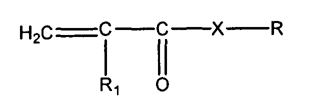 dans laquelle R₁ est H ou CH₃, R représente un groupe alkyle en C₁-C₁₀ fluoré ou non fluoré et X représente O, NH ou NR₂, où R₂ représente un groupe alkyle en C₁-C₁₀ fluoré ou non fluoré.

Selon un mode plus particulier de réalisation de l'invention, le polymère est issu d'un monomère à chaîne cristallisable choisi parmi les (méth)acrylates d'alkyle saturés en C₁₄ à C₂₂.

A titre d'exemple particulier de polymère semi-cristallin utilisable dans la composition selon l'invention, on peut citer les produits Intelimer® de la société Landec décrits dans la brochure "Intelimer® polymers", Landec IP22 (Rev. 4-97). Ces polymères sont sous forme solide à température ambiante (25°C). Ils sont porteurs de chaînes latérales cristallisables et présentent la formule X précédente.

Les polymères semi-cristallins peuvent être notamment : ceux décrits dans les exemples 3, 4, 5, 7, 9, 13 du brevet US-A-5 156 911 à groupement -COOH, résultant de la copolymérisation d'acide acrylique et d'alkyl(méth)acrylate en C₅ à de et plus particulièrement de la copolymérisation :
. d'acide acrylique, d'hexadécylacrylate et d'isodécylacrylate dans un rapport pondéral 1/16/3,
. d'acide acrylique et de pentadécylacrylate dans un rapport pondéral 1/19,
. d'acide acrylique, d'hexadécylacrylate, éthylacrylate dans un rapport pondéral 2,5/76,5/20,
. d'acide acrylique, d'hexadécylacrylate et de méthylacrylate dans un rapport pondéral 5/85/10,
. d'acide acrylique et de octadécylméthacrylate dans un rapport pondéral 2,5/97,5 ,
. d'hexadécylacrylate, de monométhyl éther de méthacrylate polyéthylèneglycol à 8 motifs d'éthylèneglycol, et d'acide acrylique dans un rapport pondéral 8,5/1/0,5.

On peut aussi utiliser le structure « O » de National Starch tel que celui décrit dans le document US-A-5 736 125 de point de fusion 44°C ainsi que les polymères semi-cristallins à chaînes pendantes cristallisables comportant des groupements fluorés tels que décrits dans les exemples 1, 4, 6, 7 et 8 du document WO-A-01/19333.

On peut également utiliser les copolymères de (méth)acrylates d'alkyles à greffons polydiméthylsiloxane tels que le copolymère de stéaryl acrylate à greffons polydiméthylsiloxane (point de fusion d'environ 30°C) ou le copolymère de béhényl acrylate à greffons polydiméthylsiloxane (point de fusion d'environ 49°C) commercialisés par la société SHIN-ETSU sous les dénominations respectives KP-561 et KP 562 (nom CTFA : acrylates/dimethicone).

On peut encore utiliser les polymères semi-cristallins de bas point de fusion obtenus par copolymérisation d'acrylate de stéaryle et d'acide acrylique ou de NVP tels que décrits dans le document US-A-5 519 063 ou EP-A-550745.
On peut aussi utiliser les polymères semi-cristallins obtenus par copolymérisation de l'acrylate de béhényle et de l'acide acrylique ou de NVP tels que décrits dans les documents US-A-5519063 et EP-A-550745.

De préférence, les polymères semi-cristallins ne comportent pas de groupement carboxyle.

La composition peut également comprendre une huile ou une huile épaissie par un agent structurant.
Dans le cas où la composition comprend des cires et/ou des polymères semi cristallins tels que décrits ci-dessus, ces derniers peuvent jouer le rôle d'agents structurants. L'agent structurant peut également être choisi parmi les gélifiants lipophiles conventionnellement utilisés en cosmétique.

Par « huile », on entend un corps gras liquide à température ambiante (25°C) et pression atmosphérique (760mm de Hg soit 105 Pa).

L'huile peut être choisi parmi toutes les huiles physiologiquement acceptables et en particulier cosmétiquement acceptables, notamment les huiles minérales, animales, végétales, synthétiques ; en particulier les huiles hydrocarbonées et/ou siliconées et/ou fluorées volatiles ou non volatiles et leurs mélanges. Plus précisément, par « huile hydrocarbonée », on entend une huile comportant principalement des atomes de carbone et d'hydrogène et éventuellement une ou plusieurs fonctions choisies parmi les fonctions hydroxyle, ester, éther, carboxylique. Généralement, l'huile présente une viscosité de 0,5 à 100 000 mPa.s, de préférence de 50 à 50 000 mPa.s et de préférence encore de 100 à 300 000 mPa.s.
A titre d'exemple d'huile utilisable dans l'invention, on peut citer :
- les huiles hydrocarbonées d'origine animale telles que le perhydrosqualène ;
- les huiles hydrocarbonées végétales telles que les triglycérides liquides d'acides gras de 4 à 24 atomes de carbone comme les triglycérides des acides heptanoïque ou octanoïque ou encore les huiles de tournesol, de maïs, de soja, de courge, de pépins de raisin, de sésame, de noisette, d'abricot, de macadamia, de ricin, d'avocat, les triglycérides des acides caprylique/caprique comme ceux vendus par la société Stearineries Dubois ou ceux vendus sous les dénominations Miglyol 810, 812 et 818 par la société Dynamit Nobel, l'huile de jojoba, de beurre de karité
- les hydrocarbures linéaires ou ramifiés, d'origine minérale ou synthétique tels que les huiles de paraffine et leurs dérivés, la vaseline, les polydécènes, les polybutènes, le polyisobutène hydrogéné tel que le Parleam ;
- les esters et les éthers de synthèse notamment d'acides gras comme les huiles de formule R₁COOR₂ dans laquelle R₁ représente le reste d'un acide gras supérieur comportant de 1 à 40 atomes de carbone et R₂ représente une chaîne hydrocarbonée contenant de 1 à 40 atomes de carbone avec R₁ + R₂ ≥ 10 comme par exemple l'huile de Purcellin, l'isononanoate d'isononyle, le myristate d'isopropyle, le palmitate d'éthyl 2-hexyle, le stéarate d'octyl 2-dodécyle, l'érucate d'octyl 2-dodécyle, l'isostéarate d'isostéaryle, le tridecyl trimellitate ; les esters hydroxylés comme l'isostéaryl lactate, l'octyl hydroxy stéarate, l'hydroxy stéarate d'octyl dodécyle, le diisostéaryl malate, le citrate de triisocétyle, des heptanoates, octanoates, décanoates d'alcools gras ; des esters de polyol comme le dioctanoate de propylène glycol, le diheptanoate de néopentyl glycol, le diisononanoate de diéthylène glycol ; et les esters du pentaérythritol comme le tétra-isostéarate de pentaérythrytyle ;
- des alcools gras ayant de 12 à 26 atomes de carbone comme l'octyl dodécanol, le 2-butyloctanol, le 2-hexyl décanol, le 2-undécyl pentadécanol, l'alcool oléique ;
- les huiles fluorées éventuellement partiellement hydrocarbonées et/ou siliconées;
- les huiles siliconées comme les polydiméthylsiloxanes (PDMS) volatiles ou non, linéaires ou cycliques ; les polydiméthylsiloxanes comportant des groupements alkyle, alcoxy ou phényle, pendant ou en bout de chaîne siliconée, groupements ayant de 2 à 24 atomes de carbone ; les silicones phénylées comme les phényl triméthicones, les phényl diméthicones, les phényl triméthylsiloxy diphényl siloxanes, les diphényl diméthicones, les diphényl méthyldiphényl trisiloxanes, les 2-phényl éthyl triméthyl-siloxysilicates,
- leurs mélanges.

A titre de gélifiants lipophiles conventionnellement utilisés en cosmétique, on peut citer par exemple les gélifiants lipophiles minéraux tels que les argiles ou les silices, les gélifiants lipophiles organiques polymériques tels que organopolysiloxanes élastomériques partiellement ou totalement réticulés, les copolymères séquencés, du type polystyrène/copoly(éthylène-propylène), les polyamides et leurs mélanges.

La composition selon l'invention peut se présenter sous forme de phase continue aqueuse ou anhydre ou sous forme d'émulsion eau-dans-huile, huile-dans-eau ou dispersion eau-dans-huile ou huile-dans-eau.

La phase grasse totale de la composition formée par la cire et/ou le polymère, en particulier le polymère semi-cristallin, et/ou l'huile épaissie par un agent structurant et un corps gras additionnel, peut être présente dans la composition selon l'invention en une teneur allant de 0,1% à 60% en poids, par rapport au poids total de la composition, de préférence allant de 0,5% à 50% en poids, et préférentiellement allant de 1 % à 40% en poids.

La composition selon l'invention peut en outre comprendre une phase aqueuse qui peut être constituée essentiellement d'eau. Elle peut comprendre également un mélange d'eau et de solvant miscible à l'eau comme les monoalcools inférieurs ayant de 1 à 5 atomes de carbone tels que l'éthanol, l'isopropanol, les glycols ayant de 2 à 8 atomes de carbone tels que le propylène glycol, l'éthylène glycol, le 1,3-butylène glycol, le dipropylène glycol, les cétones en C₃-C₄, les aldéhydes en C₂-C₄. La phase aqueuse (eau et éventuellement le solvant organique miscible à l'eau) peut représenter de 5 % à 95% en poids, par rapport au poids total de la composition.

La phase aqueuse de la composition peut être épaissie par un agent épaisissant. Parmi les agents épaississants de phase aqueuse utilisables selon l'invention, on peut citer les épaissisants cellulosiques, les argiles, les polysaccharides, les polymères acryliques, les polymères associatifs et leurs mélanges.

Dans la composition selon l'invention, la teneur en agent épaississant de phase aqueuse peut aller de 0,1 % à 15 % en poids, par rapport au poids total de la composition, de préférence de 1 % à 10 %, et mieux de 1 à 5% en poids.

La composition peut comprendre des agents tensioactifs non ioniques, anioniques, cationiques, amphotériques ou encore des émulsionnants tensioactifs. On peut se reporter au document « Encyclopedia of Chemical Technology, KIRK-OTHMER », volume 22, p. 333-432, 3^{ème} édition, 1979, WILEY, pour la définition des propriétés et des fonctions (émulsionnant) des tensioactifs, en particulier p. 347-377 de cette référence, pour les tensioactifs anioniques, amphotériques et non ioniques.

La composition selon l'invention peut également comprendre une matière colorante comme les matières colorantes pulvérulentes, les colorants liposolubles, les colorants hydrosolubles. Cette matière colorante peut être présente en une teneur allant de 0,1 % à 20 % en poids, par rapport au poids total de la composition, de préférence allant de 1 % à 15 % en poids.

Les matières colorantes pulvérulentes peuvent être choisies parmi les pigments et les nacres.

Les pigments peuvent être blancs ou colorés, minéraux et/ou organiques, enrobés ou non. On peut citer, parmi les pigments minéraux, le dioxyde de titane, éventuellement traité en surface, les oxydes de zirconium, de zinc ou de cérium, ainsi que les oxydes de fer ou de chrome, le violet de manganèse, le bleu outremer, l'hydrate de chrome et le bleu ferrique. Parmi les pigments organiques, on peut citer le noir de carbone, les pigments de type D & C, et les laques à base de carmin de cochenille, de baryum, strontium, calcium, aluminium.

Les nacres peuvent être choisies parmi les pigments nacrés blancs tels que le mica recouvert de titane ou d'oxychlorure de bismuth, les pigments nacrés colorés tels que le mica titane avec des oxydes de fer, le mica titane avec notamment du bleu ferrique ou de l'oxyde de chrome, le mica titane avec un pigment organique du type précité ainsi que les pigments nacrés à base d'oxychlorure de bismuth.

Les colorants liposolubles sont par exemple le rouge Soudan, le D&C Red 17, le D&C Green 6, le β-carotène, l'huile de soja, le brun Soudan, le D&C Yellow 11, le D&C Violet 2, le D&C orange 5, le jaune quinoléine, le rocou. Les colorants hydrosolubles sont par exemple le jus de betterave, le bleu de méthylène.

La composition selon l'invention peut comprendre en outre au moins un polymère additionnel qui est un polymère filmogène.

Le polymère filmogène peut être un polymère solubilisé ou dispersé sous forme de particules solides dans une phase aqueuse de la composition ou bien encore solubilisé ou dispersé sous forme de particules solides dans une phase grasse liquide. La composition peut comprendre un mélange de ces polymères. Lorsque le polymère filmogène se présente sous forme de particules solides, ces particules peuvent présenter une taille moyenne de particules allant de 5 nm à 600 nm, et de préférence de 20 nm à 300 nm.

Le polymère filmogène peut être présent dans la composition selon l'invention en une teneur en matières sèches allant de 0,1 % à 60 % en poids par rapport au poids total de la composition, de préférence de 0,5 % à 40 % en poids, et mieux de 1 % à 30 % en poids.

Dans la présente demande, on entend par "polymère filmogène", un polymère apte à former à lui seul ou en présence d'un agent auxiliaire de filmification, un film continu et adhérent sur un support, notamment sur les matières kératiniques.

Parmi les polymères filmogènes utilisables dans la composition de la présente invention, on peut citer les polymères synthétiques, de type radicalaire ou de type polycondensat, les polymères d'origine naturelle, et leurs mélanges.

Par polymère filmogène radicalaire, on entend un polymère obtenu par polymérisation de monomères à insaturation notamment éthylénique, chaque monomère étant susceptible de s'homopolymériser (à l'inverse des polycondensats).
Les polymères filmogènes de type radicalaire peuvent être notamment des polymères, ou des copolymères, vinyliques, notamment des polymères acryliques.

Les polymères filmogènes vinyliques peuvent résulter de la polymérisation de monomères à insaturation éthylénique ayant au moins un groupement acide et/ou des esters de ces monomères acides et/ou des amides de ces monomères acides.

Comme monomère porteur de groupement acide, on peut utiliser des acides carboxyliques insaturés α,β-éthyléniques tels que l'acide acrylique, l'acide méthacrylique, l'acide crotonique, l'acide maléique, l'acide itaconique. On utilise de préférence l'acide (méth)acrylique et l'acide crotonique, et plus préférentiellement l'acide (méth)acrylique.

Les esters de monomères acides sont avantageusement choisis parmi les esters de l'acide (méth)acrylique (encore appelé les (méth)acrylates), notamment des (méth)acrylates d'alkyle, en particulier d'alkyle en C₁-C₃₀, de préférence en C₁-C₂₀, des (méth)acrylates d'aryle, en particulier d'aryle en C₆-C₁₀, des (méth)acrylates d'hydroxyalkyle, en particulier d'hydroxyalkyle en C₂-C₆.
Parmi les (méth)acrylates d'alkyle, on peut citer le méthacrylate de méthyle, le méthacrylate d'éthyle, le méthacrylate de butyle, le méthacrylate d'isobutyle, le méthacrylate d'éthyl-2 hexyle, le méthacrylate de lauryle, le méthacrylate de cyclohexyle.
Parmi les (méth)acrylates d'hydroxyalkyle, on peut citer l'acrylate d'hydroxyéthyle, l'acrylate de 2-hydroxypropyle, le méthacrylate d'hydroxyéthyle, le méthacrylate de 2-hydroxypropyle.
Parmi les (méth)acrylates d'aryle, on peut citer l'acrylate de benzyle et l'acrylate de phényle.
Les esters de l'acide (méth)acrylique particulièrement préférés sont les (méth)acrylates d'alkyle.

Le groupement alkyle des esters peut être soit fluoré, soit perfluoré, c'est-à-dire qu'une partie ou la totalité des atomes d'hydrogène du groupement alkyle sont substitués par des atomes de fluor.

Comme amides des monomères acides, on peut par exemple citer les (méth)acrylamides, et notamment les N-alkyl (méth)acrylamides, en particulier d'alkyl en C₂-C₁₂. Parmi les N-alkyl (méth)acrylamides, on peut citer le N-éthyl acrylamide, le N-t-butyl acrylamide, le N-t-octyl acrylamide et le N-undécylacrylamide.

Les polymères filmogènes vinyliques peuvent également résulter de l'homopolymérisation ou de la copolymérisation de monomères choisis parmi les esters vinyliques et les monomères styrèniques. En particulier, ces monomères peuvent être polymérisés avec des monomères acides et/ou leurs esters et/ou leurs amides, tels que ceux mentionnés précédemment.
Comme exemple d'esters vinyliques, on peut citer l'acétate de vinyle, le néodécanoate de vinyle, le pivalate de vinyle, le benzoate de vinyle et le t-butyl benzoate de vinyle.
Comme monomères styrèniques, on peut citer le styrène et l'alpha-méthyl styrène.

Il est possible d'utiliser tout monomère connu de l'homme du métier entrant dans les catégories de monomères acryliques et vinyliques (y compris les monomères modifiés par une chaîne siliconée).

Parmi les polycondensats filmogènes, on peut citer les polyuréthanes, les polyesters, les polyesters amides, les polyamides, et les résines époxyesters, les polyurées.

Les polyuréthanes peuvent être choisis parmi les polyuréthanes anioniques, cationiques, non-ioniques ou amphotères, les polyuréthanes-acryliques, les polyuréthanes-polyvinylpirrolidones, les polyester-polyuréthanes, les polyéther-polyuréthanes, les polyurées, les polyurée-polyuréthanes, et leurs mélanges.

Les polyesters peuvent être obtenus, de façon connue, par polycondensation d'acides dicarboxyliques avec des polyols, notamment des diols.
L'acide dicarboxylique peut être aliphatique, alicyclique ou aromatique. On peut citer comme exemple de tels acides : l'acide oxalique, l'acide malonique, l'acide diméthylmalonique, l'acide succinique, l'acide glutarique, l'acide adipique, l'acide pimélique, l'acide 2,2-diméthylglutarique, l'acide azélaïque, l'acide subérique, l'acide sébacique, l'acide fumarique, l'acide maléique, l'acide itaconique, l'acide phtalique, l'acide dodécanedioïque, l'acide 1,3-cyclohexanedicarboxylique, l'acide 1,4-cyclohexa-nedicarboxylique, l'acide isophtalique, l'acide téréphtalique, l'acide 2,5-norbornane dicarboxylique, l'acide diglycolique, l'acide thiodipropionique, l'acide 2,5-naphtalènedicarboxylique, l'acide 2,6-naphtalènedicarboxylique. Ces monomères acide dicarboxylique peuvent être utilisés seuls ou en combinaison d'au moins deux monomères acide dicarboxylique. Parmi ces monomères, on choisit préférentiellement l'acide phtalique, l'acide isophtalique, l'acide téréphtalique.

Le diol peut être choisi parmi les diols aliphatiques, alicycliques, aromatiques. On utilise de préférence un diol choisi parmi : l'éthylène glycol, le diéthylène glycol, le triéthylène glycol, le 1,3-propanediol, le cyclohexane diméthanol, le 4-butanediol.
Comme autres polyols, on peut utiliser le glycérol, le pentaérythritol, le sorbitol, le triméthylol propane.

Les polyesters amides peuvent être obtenus de manière analogue aux polyesters, par polycondensation de diacides avec des diamines ou des amino alcools.
Comme diamine, on peut utiliser l'éthylènediamine, l'hexaméthylènediamine, la méta- ou para-phénylènediamine. Comme aminoalcool, on peut utiliser la monoéthanolamine.

Le polyester peut en outre comprendre au moins un monomère portant au moins un groupement -SO₃M, avec M représentant un atome d'hydrogène, un ion ammonium NH₄⁺ ou un ion métallique, comme par exemple un ion Na⁺, Li⁺, K+, Mg²⁺, Ca²⁺, Cu²⁺, Fe²⁺, Fe³⁺. On peut utiliser notamment un monomère aromatique bifonctionnel comportant un tel groupement -SO₃M.

Le noyau aromatique du monomère aromatique bifonctionnel portant en outre un groupement -SO₃M tel que décrit ci-dessus peut être choisi par exemple parmi les noyaux benzène, naphtalène, anthracène, diphényl, oxydiphényl, sulfonyldiphényl, méthylènediphényl. On peut citer comme exemple de monomère aromatique bifonctionnel portant en outre un groupement -SO₃M : l'acide sulfoisophtalique, l'acide sulfotéréphtalique, l'acide sulfophtalique, l'acide 4-sulfonaphtalène-2,7-dicarboxylique.
On préfère utiliser des copolymères à base d'isophtalate/sulfoisophtalate, et plus particulièrement des copolymères obtenus par condensation de di-éthylèneglycol, cyclohexane di-méthanol, acide isophtalique, acide sulfoisophtalique. De tels polymères sont vendus par exemple sous le nom de marque Eastman AQ® par la société Eastman Chemical Products.

Les polymères d'origine naturelle, éventuellement modifiés, peuvent être choisis parmi la résine shellac, la gomme de sandaraque, les dammars, les élémis, les copals, les polymères cellulosiques, et leurs mélanges.

Selon un premier mode de réalisation de la composition selon l'invention, le polymère filmogène peut être présent sous la forme de particules en dispersion aqueuse, connue généralement sous le nom de latex ou pseudolatex. Les techniques de préparation de ces dispersions sont bien connues de l'homme du métier.

Comme dispersion aqueuse de polymère filmogène, on peut utiliser les dispersions acryliques vendues sous les dénominations NEOCRYL XK-90® , NEOCRYL A-1070® , NEOCRYL A-1090® , NEOCRYL BT-62® , NEOCRYL A-1079® , NEOCRYL A-523® par la société AVECIA-NEORESINS, DOW LATEX 432® par la société DOW CHEMICAL, DAITOSOL 5000 AD® par la société DAITO KASEY KOGYO; SYNTRAN 5760, SYNTRAN 5190 et SYNTRAN 5170 commercialisés par la société INTERPOLYMER ou bien encore les dispersions aqueuses de polyuréthane vendues sous les dénominations NEOREZ R-981® , NEOREZ R-974® par la société AVECIA-NEORESINS, les AVALURE UR-405® , AVALURE UR-410® , AVALURE UR-425® , AVALURE UR-450® , SANCURE 875 ® , SANCURE 861® , SANCURE 878® , SANCURE 2060® par la société GOODRICH, IMPRANIL 85® par la société BAYER, AQUAMERE H-1511® par la société HYDROMER,

Comme dispersion aqueuse de polymère filmogène, on peut également utiliser les dispersions de polymères résultant de la polymérisation radicalaire d'un ou plusieurs monomères radicalaires à l'intérieur et/ou partiellement en surface, de particules préexistantes d'au moins un polymère choisi dans le groupe constitué par les polyuréthanes, les polyurées, les polyesters, les polyesteramides et/ou les alkydes. Ces polymères sont généralement appelés polymères hybrides.

Selon une deuxième variante de réalisation de la composition selon l'invention, le polymère filmogène peut être un polymère hydrosoluble et est donc présent dans la phase aqueuse de la composition sous forme solubilisée. Comme exemples de polymères filmogènes hydrosolubles, on peut citer
- les protéines comme les protéines d'origine végétale telles que les protéines de blé, de soja ; les protéines d'origine animale tels que les kératines, par exemples les hydrolysats de kératine et les kératines sulfoniques ;
- les polymères de chitine ou de chitosane anioniques, cationiques, amphotères ou non-ioniques ;
- les polymères de cellulose tels que l'hydroxyéthylcellulose, l'hydroxypropylcellulose, la méthylcellulose, l'éthylhydroxyéthylcellulose, la carboxyméthylcellulose, ainsi que les dérivés quaternisés de la cellulose ;
- les polymères ou copolymères acryliques, tels que les polyacrylates ou les polyméthacrylates, par exemple le polyméthacrylate de sodium fabriqué ou commercialisé par la société VANDERBILT sous la référence commerciale DARVAN 7;
- les polymères vinyliques, comme les polyvinylpyrrolidones, les copolymères de l'éther méthylvinylique et de l'anhydride malique, le copolymère de l'acétate de vinyle et de l'acide crotonique, les copolymères de vinylpyrrolidone et d'acétate de vinyle ; les copolymères de vinylpyrrolidone et de caprolactame ; l'alcool polyvinylique ;
- les polymères d'origine naturelle, éventuellement modifiés, tels que :
   . les gommes arabiques, la gomme de guar, les dérivés du xanthane, la gomme de karaya ;
   . les alginates et les carraghénanes ;
   . les glycoaminoglycanes, l'acide hyaluronique et ses dérivés ;
   . la résine shellac, la gomme de sandaraque, les dammars, les élémis, les copals ;
   . l'acide désoxyribonucléïque ;
   . les muccopolysaccharides tels que l'acide hyaluronique, les chondroïtines sulfate,
et leurs mélanges.

Selon une autre variante de réalisation de la composition selon l'invention, le polymère filmogène peut être présent dans une phase grasse liquide comprenant des huiles ou solvants organiques. Par "phase grasse liquide", on entend, au sens de l'invention, une phase grasse liquide à température ambiante (25°C) et pression atmosphérique (760 mm de Hg, soit 105 Pa), composée d'un ou plusieurs corps gras liquides à température ambiante, appelés aussi huiles, généralement compatibles entre eux.
De préférence, la phase grasse liquide comprend une huile volatile, éventuellement en mélange avec une huile non volatile, les huiles pouvant être choisies parmi les huiles citées précédemment.

Selon un autre de réalisation de la composition selon l'invention, le polymère filmogène peut être présent sous forme de particules, stabilisées en surface, dispersées dans la phase grasse liquide.

La dispersion de particules de polymère stabilisées en surface peut être fabriquée comme décrit dans le document EP-A-749747.

Les particules de polymère sont stabilisées en surface grâce à un stabilisant qui peut être un polymère séquencé, un polymère greffé, et/ou un polymère statistique, seul ou en mélange.

Des dispersions de polymère filmogène dans la phase grasse liquide, en présence d'agent stabilisants, sont notamment décrites dans les documents EP-A-749746, EP-A-923928, EP-A-930060 dont le contenu est incorporé à titre de référence dans la présente demande.

La taille des particules de polymères en dispersion soit dans la phase aqueuse, soit dans la phase grasse liquide, peut aller de 5 nm à 600 nm, et de préférence de 20 nm à 300 nm.

Selon un quatrième mode de réalisation de la composition selon l'invention, le polymère filmogène peut être solubilisé dans la phase grasse liquide, on dit alors que le polymère filmogène est un polymère liposoluble.

A titre d'exemple de polymère liposoluble, on peut citer les copolymères d'ester vinylique (le groupe vinylique étant directement relié à l'atome d'oxygène du groupe ester et l'ester vinylique ayant un radical hydrocarboné saturé, linéaire ou ramifié, de 1 à 19 atomes de carbone, lié au carbonyle du groupe ester ) et d'au moins un autre monomère qui peut être un ester vinylique (différent de l'ester vinylique déjà présent), une α-oléfine (ayant de 8 à 28 atomes de carbone), un alkylvinyléther (dont le groupe alkyl comporte de 2 à 18 atomes de carbone), ou un ester allylique ou méthallylique (ayant un radical hydrocarboné saturé, linéaire ou ramifié, de 1 à 19 atomes de carbone, lié au carbonyle du groupe ester).

Ces copolymères peuvent être réticulés à l'aide de réticulants qui peuvent être soit du type vinylique, soit du type allylique ou méthallylique, tels que le tétraallyloxyéthane, le divinylbenzène, l'octanedioate de divinyle, le dodécanedioate de divinyle, et l'octadécanedioate de divinyle.

Comme exemples de ces copolymères, on peut citer les copolymères : acétate de vinyle/stéarate d'allyle, l'acétate de vinyle/laurate de vinyle, acétate de vinyle/stéarate de vinyle, acétate de vinyle/octadécène, acétate de vinyle/octadécylvinyléther, propionate de vinyle/laurate d'allyle, propionate de vinyle/laurate de vinyle, stéarate de vinyle/octadécène-1, acétate de vinyle/dodécène-1, stéarate de vinyle/éthylvinyléther, propionate de vinyle/cétyl vinyle éther, stéarate de vinyle/acétate d'allyle, diméthyl-2, 2 octanoate de vinyle/laurate de vinyle, diméthyl-2, 2 pentanoate d'allyle/laurate de vinyle, diméthyl propionate de vinyle/stéarate de vinyle, diméthyl propionate d'allyle/stéarate de vinyle, propionate de vinyle/stéarate de vinyle, réticulé avec 0,2 % de divinyl benzène, diméthyl propionate de vinyle/laurate de vinyle, réticulé avec 0,2 % de divinyl benzène, acétate de vinyle/octadécyl vinyl éther, réticulé avec 0,2 % de tétraallyloxyéthane, acétate de vinyle/stéarate d'allyle, réticulé avec 0,2 % de divinyl benzène, acétate de vinyle/octadécène-1 réticulé avec 0,2 % de divinyl benzène et propionate d'allyle/stéarate d'allyle réticulé avec 0,2 % de divinyl benzène.

Comme polymères filmogènes liposolubles, on peut également citer les homopolymères liposolubles, et en particulier ceux résultant de l'homopolymérisation d'esters vinyliques ayant de 9 à 22 atomes de carbone ou d'acrylates ou de méthacrylates d'alkyle, les radicaux alkyles ayant de 10 à 20 atomes de carbone.

De tels homopolymères liposolubles peuvent être choisis parmi le polystéarate de vinyle, le polystéarate de vinyle réticulé à l'aide de divinylbenzène, de diallyléther ou de phtalate de diallyle, le poly(méth)acrylate de stéaryle, le polylaurate de vinyle, le poly(méth)acrylate de lauryle, ces poly(méth)acrylates pouvant être réticulés à l'aide de diméthacrylate de l'éthylène glycol ou de tétraéthylène glycol.

Les copolymères et homopolymères liposolubles définis précédemment sont connus et notamment décrits dans la demande FR-A-2262303 ; ils peuvent avoir un poids moléculaire moyen en poids allant de 2.000 à 500.000 et de préférence de 4.000 à 200.000.

Comme polymères filmogènes liposolubles utilisables dans l'invention, on peut également citer les polyalkylènes et notamment les copolymères d'alcènes en C₂-C₂₀, comme le polybutène, les alkylcelluloses avec un radical alkyle linéaire ou ramifié, saturé ou non en C₁ à C₈ comme l'éthylcellulose et la propylcellulose, les copolymères de la vinylpyrolidone (VP) et notamment les copolymères de la vinylpyrrolidone et d'alcène en C₂ à C₄₀ et mieux en C₃ à C₂₀. A titre d'exemple de copolymère de VP utilisable dans l'invention, on peut citer le copolymère de VP/acétate vinyle, VP/méthacrylate d'éthyle, la polyvinylpyrolidone (PVP) butylée, VP/méthacrylate d'éthyle/acide méthacrylique, VP/eicosène, VP/hexadécène,

VP/triacontène, VP/styrène, VP/acide acrylique/méthacrylate de lauryle.

La composition selon l'invention peut comprendre un agent auxiliaire de filmification favorisant la formation d'un film avec le polymère filmogène. Un tel agent de filmification peut être choisi parmi tous les composés connus de l'homme du métier comme étant susceptibles de remplir la fonction recherchée, et notamment être choisi parmi les agents plastifiants et les agents de coalescence.

La composition de l'invention peut comprendre, en outre, tout additif usuellement utilisé en cosmétique tels que les antioxydants, les conservateurs, les parfums, les neutralisants, les plastifiants, les actifs cosmétiques comme par exemple des émollients, des hydratants, des vitamines, des filtres solaires, et leurs mélanges. Ces additifs peuvent être présents dans la composition en une teneur allant de 0,01 à 10 %, du poids total de la composition.

Bien entendu l'homme du métier veillera à choisir les éventuels additifs complémentaires et/ou leur quantité de telle manière que les propriétés avantageuses de la composition selon l'invention ne soient pas altérées par l'adjonction envisagée, et notamment de manière que le profil thermique de la composition reste tel que défini plus haut.

La composition selon l'invention peut être fabriquée par les procédés connus, généralement utilisés dans le domaine cosmétique.

La composition selon l'invention est destinée de préférence à être chauffée.

Le chauffage de la composition peut être mis en oeuvre préalablement à l'application du produit. A cet effet, le réservoir contenant la composition peut, à la manière de ce qui est décrit dans le brevet US 5 775 344, être équipé de moyens de chauffage disposés sur les parois intérieures du réservoir de manière à chauffer le produit qu'il contient.

Alternativement, le chaufffage peut se faire au micro-ondes ou au bain marie, en utilisant un dispositif du type de celui décrit dans le brevet US 5 856 653.

Le chauffage de la composition, notamment en vue de recourber les cils, peut être réalisé après l'application du produit, au moyen par exemple de dispositifs tels que décrits dans le brevet US 5 853 010.

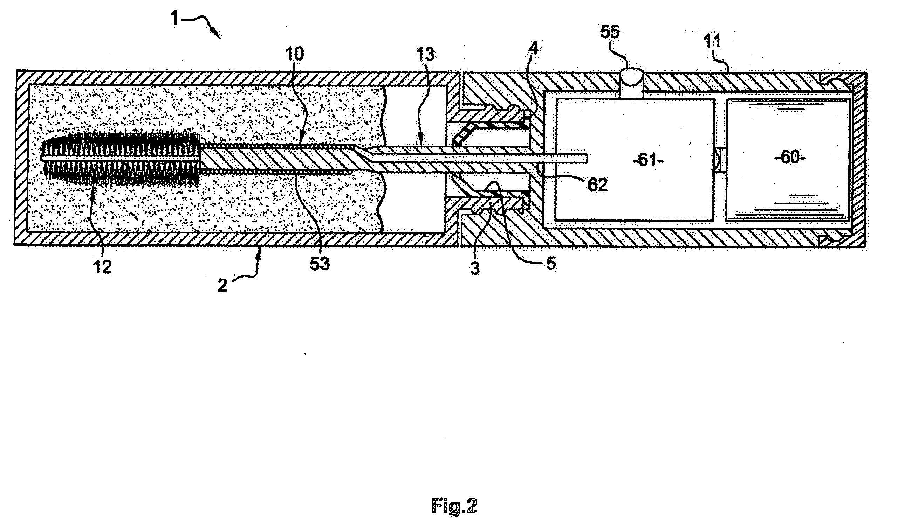

Avantageusement toutefois, dans le cas où la composition selon l'invention est un mascara pour les cils, l'application et le chauffage se font de manière combinée au moyen du dispositif tel que représenté à la figure 2.

Un tel dispositif comprend un récipient 2 conforme au récipient 2 de la figure 2.

A l'intérieur du capuchon 11 est disposé une source d'alimentation en courant continu 60, notamment sous forme d'une batterie rechargeable, reliée à une électronique de régulation 61. Un interrupteur sous forme d'un poussoir 55 permet sélectivement d'activer/désactiver la partie chauffante 53 du dispositif d'application 10. Une LED est intégrée au poussoir 55 et change de couleur lorsque la composition contenue dans le réservoir 2 est à la température adéquate.

Le capuchon 11 est solidaire d'une tige 13 à extrémité de laquelle est fixée une brosse 12 de type brosse torsadée.

L'électronique de régulation est reliée à une résistance 53 configurée sous forme d'un fil enroulé sur la surface extérieure de la tige 13. L'enroulement est à spires jointives et s'étend sensiblement de l'extrémité de la tige adjacente à la brosse 12, à une portion de la tige située sensiblement au niveau de la surface libre du produit à l'intérieur du récipient préalablement à la première utilisation du dispositif. La partie de la tige située au dessus de l'enroulement hélicoïdal n'est pas chauffée de manière sensible. C'est notamment le cas de la portion de la tige située en regard de la lèvre de l'organe d'essorage 5.

Entre la partie enroulée sur la tige 13 (résistance 53) et le circuit de régulation 61, les fils 62 passent à l'intérieur de la tige 13. Leur diamètre (de l'ordre du mm) est sensiblement supérieur à celui de la résistance chauffante 53, de manière à ce qu'il ne soient pas chauffés de manière sensible sur cette portion. On réduit ainsi au maximum l'inertie du système. De même, du fait de l'absence sensible de chauffage de la tige en regard de l'essoreur, ce dernier peut être réalisé en matériau conventionnel.

Ainsi, l'utilisatrice au moyen de l'interrupteur 55 active le chauffage de la résistance 53. Cette dernière est au contact du produit à l'intérieur du récipient 2 et le porte à une température supérieure à sa température de fin de fusion. Typiquement, la composition est chauffée à environ 60°.

Lorsque la température est atteinte, la LED de l'interrupteur 55 passe au vert. L'utilisatrice dévisse alors le capuchon 11, et extrait l'applicateur du récipient 2.

Lors du mouvement d'extraction, la brosse 12 est essorée de manière conventionnelle. Le produit ainsi chauffé est alors appliqué de manière conventionnelle, en imprimant aux cils un mouvement destiné à les recourber.

En refroidissant, la composition revient à son état cristallin, et ceci de manière très rapide en raison de la faible largeur du pic de fusion. Les cils sont figés dans leur configuration recourbée recherchée et ceci de façon durable.

Alternativement, les moyens de chauffage sont réalisés sous forme notamment d'une lampe à incandescence et sont disposés à l'intérieur d'un élément creux dont la surface extérieure comporte des éléments d'applications, notamment des stries, des dents, ou des poils.

L'invention est illustrée plus en détail dans les exemples suivants.

### Exemple 1 :

On a préparé un mascara ayant la composition suivante :
- Polymère semi-cristallin (polyacrylate de stéaryle) 23,3 g
- Polybutène 11,7 g
- Acide stéarique 5,8 g
- Aminopropanediol 0,5 g
- Hydroxyéthylcellulose 0,9 g
- Gomme arabique 3,45 g
- Triéthanolamine 2,4 g
- Pigments 8 g
- Conservateurs qs
- Eau qsp 100 g

### Mode opératoire

Le polymère semi-cristallin et le polybutène sont chauffés jusqu'à fusion puis placés sous agitation magnétique.
L'acide stéarique, l'aminopropanediol et les pigments sont ajoutés et l'ensemble est chauffé au bain-marie sous agitation à l'aide d'un agitateur de Moritz.
L'eau, les conservateurs, l'hydroxyéthylcellulose, la gomme arabique et la triéthanolamine sont ensuite ajoutés sous agitation rapide pour réaliser l'émulsion.

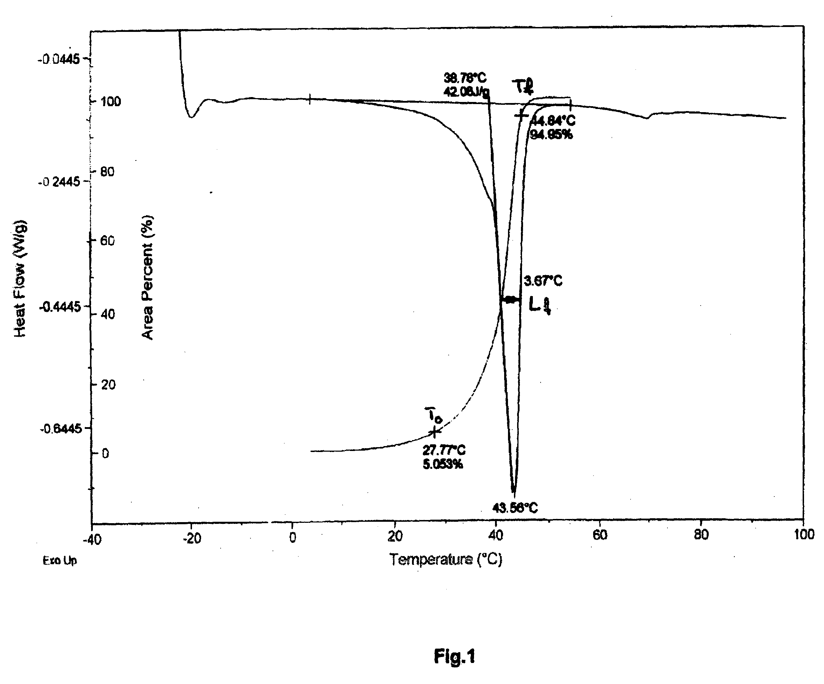

Cette composition de mascara présente un profil thermique caractérisé par un pic de fusion tel que représenté en figure 1.
Il présente les paramètres suivants :
- température de début de fusion To = 27,77°C
- température de fin de fusion Tf = 44,84 °C
- amplitude de fusion ΔT = 17,07°C
- largeur de pic à mi hauteur Lf = 3,67°C
Le point de fusion de la composition est de 43,56°C.

La stabilité thermique de la composition est déterminée de la manière suivante :
- on mesure la viscosité de la composition à To,
- la composition est placée dans une étuve à 80°C pendant 2 heures puis on la laisse revenir à température ambiante après sortie de l'étuve (ce qui correspond à un cycle de fusion). L'opération est répétée plusieurs fois et la viscosité est mesurée à la fin de chaque cycle à l'aide d'un Rhéomat RM 180 équipé d'un mobile MS-r3 ou Ms-r4 tournant à 240 min⁻¹ pour une alimentation en courant à 60 Hz ou à 200 min⁻¹ pour une alimentation en courant à 50 Hz.

On obtient les résultats suivants :

| | à To | Après 1 cycle de fusion | Après 2 cycles de fusion | Après 3 cycles de fusion | Après 4 cycles de fusion | Après 10 cycles de fusion | Après 15 cycles de fusion |
|---|---|---|---|---|---|---|---|
| Viscosité (Pa.s) | 6,7 | 7,4 | 7,2 | 7,7 | 7,6 | 7,2 | 7,1 |
| Variation de la viscosité en % | | 10 | 7,5 | 14,9 | 13,4 | 7,5 | 5,9 |

On constate que cette composition est apte à retrouver sa viscosité initiale ± 15% après plusieurs cycles de chauffage à une température supérieure à son point de fusion.

Après application de la composition sur les cils puis chauffage du film de composition quelques secondes à l'aide d'une brosse chauffante, ce mascara a été jugé comme permettant d'améliorer significativement le recourbement des cils.

## Revendications

1. Composition cosmétique comprenant :
i) un milieu physiologiquement acceptable et
ii) au moins un composé conférant à la composition un profil thermique présentant un pic de fusion dont la largeur à mi-hauteur Lf est inférieure ou égale à 20 °C,
ladite composition étant stable thermiquement.

2. Composition selon la revendication précédente, **caractérisée en ce qu'**elle présente une température de début de fusion To supérieure ou égale à 10°C, de préférence supérieure ou égale à 15°C et mieux supérieure ou égale à 20°C.

3. Composition selon l'une des revendications précédentes, **caractérisée en ce qu'**elle présente une température de fin de fusion Tf inférieure ou égale à 90°C, mieux inférieure ou égale à 80°C, et mieux encore, inférieure ou égale à 70°C.

4. Composition selon l'une des revendications précédentes, **caractérisée en ce que** le pic de fusion présente un point de fusion Pf allant de 20°C à 80°C.

5. Composition selon l'une des revendications précédentes, **caractérisée en ce que** le pic de fusion présente une largeur à mi-hauteur Lf inférieure ou égale à 10°C.

6. Composition selon l'une des revendications précédentes, **caractérisée en ce que** le pic de fusion présente une amplitude de température ΔT = Tf - To inférieure ou égale à 30°C.

7. Composition selon l'une des revendications précédentes, **caractérisée en ce que** ledit composé a un profil thermique présentant un pic de fusion dont la largeur à mi-hauteur Lf est inférieure ou égale à 20 °C.

8. Composition selon l'une des revendications précédentes, **caractérisée en ce que** ledit composé est choisi parmi les cires, les polymères, de préférence les polymères semi-cristallins, les huiles épaissies par un agent structurant et leurs mélanges.

9. Composition selon l'une des revendications précédentes, **caractérisée en ce que** ledit composé est présente en une teneur allant de 1% à 60%, de préférence de 3% à 55%, mieux de 5% à 50% et encore mieux de 10 à 40% en poids par rapport au poids total de la composition.

10. Composition selon l'une des revendications précédentes **caractérisée en ce qu'**elle comprend un polymère semi-cristallin choisi parmi les copolymères résultant de la polymérisation d'au moins un monomère à chaîne cristallisable choisi parmi les (méth)acrylates d'alkyle saturés en C₁₄ à C₂₄, les (méth)acrylates de perfluoroalkyle en C₁₁ à C₁₅, les N alkyl (méth)acrylamides en C₁₄ à C₂₄ avec ou sans atome de fluor, les esters vinyliques à chaînes alkyle ou perfluoroalkyle en C₁₄ à C₂₄, les éthers vinyliques à chaînes alkyle ou perfluoroalkyle en C₁₄ à C₂₄, les alphaoléfines en C₁₄ à C₂₄, les para-alkyl styrènes avec un groupe alkyle comportant de 12 à 24 atomes de carbone, avec au moins un ester ou amide d'acide monocarboxylique en C₁ à C₁₀ fluoré ou non fluoré, de formule suivante : 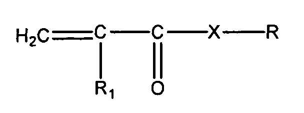 dans laquelle R₁ est H ou CH₃, R représente un groupe alkyle en C₁-C₁₀ fluoré ou non fluoré et X représente O, NH ou NR₂, où R₂ représente un groupe alkyle en C₁-C₁₀fluoré ou non fluoré.

11. Composition selon l'une des revendications précédentes, **caractérisée en ce qu'**elle comprend une cire choisie parmi la cire d'olive obtenue par hydrogénation d'huile d'olive estérifiée avec l'alcool stéarylique, l'alcool stéarique, le stéarate de stéaryle, le benzoate de stéaryle, le ditrimethylolpropanetetrastéarate, les acides gras de cire de Montan polyéthoxylés, le ditriméthylolpropanetetrabéhénate, la cire de dioctadécylcarbonate et leurs mélanges.

12. Composition selon l'une des revendications précédentes, **caractérisée en ce qu'**elle comprend une huile épaissie par un agent structurant.

13. Composition selon l'une des revendications précédentes, **caractérisée en ce qu'**elle comprend une phase aqueuse.

14. Composition selon la revendication précédente, **caractérisée en ce que** la phase aqueuse représente de 5 % à 95 % en poids, par rapport au poids total de la composition.

15. Composition selon la revendication 13 ou 14, **caractérisée en ce que** la phase aqueuse est épaissie par un agent épaississant.

16. Composition selon l'une des revendications précédentes, **caractérisée en ce qu'**elle comprend un polymère filmogène.

17. Composition selon l'une des revendications précédentes, **caractérisée en ce que** le polymère filmogène est présent en une teneur en matières sèches allant de 0,1 % à 60 % en poids par rapport au poids total de la composition, de préférence de 0,5 % à 40 % en poids, et mieux de 1 % à 30 % en poids.

18. Composition selon l'une des revendications précédentes, **caractérisée en ce qu'**elle comprend une matière colorante.

19. Composition selon l'une des revendications précédentes, **caractérisée en ce que** la matière colorante est présente en une teneur allant de 0,1 % à 20 % en poids, par rapport au poids total de la composition, de préférence allant de 1 % à 15 % en poids.

20. Composition selon l'une des revendications précédentes, **caractérisée en ce qu'**elle est une composition de revêtement des fibres kératiniques.

21. Composition cosmétique comprenant :
i) un milieu physiologiquement acceptable et
ii) au moins un polymère conférant à ladite composition un profil thermique présentant un pic de fusion dont la largeur à mi-hauteur Lf est inférieure ou égale à 10 °C.

22. Composition selon la revendication 21 **caractérisée en ce qu'**elle est stable thermiquement.

23. Composition selon la revendication 21 ou 22, **caractérisée en ce que** le polymère est un polymère semi-cristallin.

24. Procédé cosmétique de maquillage ou de soin non thérapeutique des matières kératiniques comprenant l'application sur les matières kératiniques d'une composition cosmétique selon l'une quelconque des revendications qui précèdent, ladite composition étant, antérieurement, simultanément, ou postérieurement à son application, portée à une température supérieure ou égale à son point de fusion, de préférence supérieure ou égale à sa température de fin de fusion.

25. Procédé selon la revendication 24, **caractérisé en ce que** la composition est portée à une température supérieure ou égale à son point de fusion préalablement à son application sur les matières kératiniques, notamment par chauffage au micro-ondes ou au bain-marie, ou par tout autre moyen de chauffage équipant un ensemble de conditionnement et d'application la contenant, en particulier, les parois d'un réservoir contenant la composition.

26. Procédé selon la revendication 24, **caractérisé en ce que** la composition est portée à une température supérieure ou égale à son point de fusion simultanément ou postérieurement à son application sur les matières kératiniques, notamment à l'aide d'un dispositif d'application comprenant des moyens de chauffage.

27. Utilisation d'une composition cosmétique de revêtement des fibres kératiniques, ladite composition comprenant :
i) un milieu physiologiquement acceptable et
ii) au moins un composé conférant à ladite composition un profil thermique présentant un pic de fusion dont la largeur à mi-hauteur Lf est inférieure ou égale à 20 °C,
ladite composition étant stable thermiquement, pour obtenir un film déposé sur les fibres kératiniques homogène et/ou présentant des propriétés de recourbement améliorées.
